# EUROPEAN PATENT APPLICATION

(11) **EP 0 915 158 A2**
(43) Date of publication of application: **12.05.1999**
(21) Application number: 98203646.9
(22) Date of filing: 28.10.1998
(51) Int. Cl.: C12N 15/13, C07K 16/12, C12N 5/20, A61K 39/112, G01N 33/569, G01N 33/577, C07K 14/255

(54) **Improvements in or relating to detection of salmonella**

(30) Priority: 04.11.1997 EP 97308838
(71) Applicant: UNILEVER N.V., 3000 DK Rotterdam (NL); UNILEVER PLC, London EC4P 4BQ (GB)
(72) Inventor: Berry, Mark John, Sharnbrook, Bedford MK44 1LQ (GB); Metcalfe, Mark Andrew, Southampton, Hampshire SO18 1LZ (GB); Parry, Steven H., Sharnbrook, Bedford MK44 1LQ (GB)
(74) Representative: Rosen Jacobson, Frans Lucas M.

(57) **Abstract**

Disclosed is an immunoglobulin molecule or antigen-binding variant thereof which binds to an epitope present within amino acid residues 403 to 452 of the flagellin protein of *Salmonella typhimurium* PIR accession No. A24262, or the equivalent portion from other *Salmonella* strains together with, inter alia, use thereof, and an assay device comprising the antibody.

SEQUENCE LISTING

(1) GENERAL INFORMATION:

(i) APPLICANT:
(A) NAME: Unilever PLC
(B) STREET: Unilever House, Blackfriars
(C) CITY: London
(E) COUNTRY: United Kingdom
(F) POSTAL CODE (ZIP): EC4P 4BQ
(G) TELEPHONE: (01234) 781781
(H) TELEFAX: (01234) 222633

(ii) TITLE OF INVENTION: Improvements in or Relating to
Detection of Salmonella

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 50 amino acids
(B) TYPE: amino acid
(C) STRANDEDNESS:
(D) TOPOLOGY: linear

(ii) MOLECULE TYPE: peptide

(v) FRAGMENT TYPE: internal

(vi) ORIGINAL SOURCE:
(A) ORGANISM: Salmonella typhimurium

(xi) SEQUENCE DESCRIPTION: SEQ ID NO: 1:

Pro Leu Ala Lys Ile Asp Ala Ala Leu Ala Gln Val Asp Ala Leu Arg
1               5                   10                  15

Ser Asp Leu Gly Ala Val Gln Asn Arg Phe Asp Ser Ala Ile Thr Asn
            20                  25                  30

Leu Gly Asn Thr Val Asn Asn Leu Ser Ser Ala Arg Ser Arg Ile Glu
        35                  40                  45

Asp Ala
    50

(2) INFORMATION FOR SEQ ID NO: 2:

(i) SEQUENCE CHARACTERISTICS:
(A) LENGTH: 10 amino acids
(B) TYPE: amino acid
(C) STRANDEDNESS:
(D) TOPOLOGY: linear

(ii) MOLECULE TYPE: peptide

(v) FRAGMENT TYPE: internal

(xi) SEQUENCE DESCRIPTION: SEQ ID NO: 2:

Arg Phe Asp Ser Ala Ile Thr Asn Leu Gly
1               5                   10

(2) INFORMATION FOR SEQ ID NO: 3:

(i) SEQUENCE CHARACTERISTICS:
(A) LENGTH: 8 amino acids
(B) TYPE: amino acid
(C) STRANDEDNESS:
(D) TOPOLOGY: linear

(ii) MOLECULE TYPE: peptide

(v) FRAGMENT TYPE: internal

(xi) SEQUENCE DESCRIPTION: SEQ ID NO: 3:

Ser Ala Arg Ser Arg Ile Glu Asp
1               5

(2) INFORMATION FOR SEQ ID NO: 4:

(i) SEQUENCE CHARACTERISTICS:
(A) LENGTH: 22 amino acids
(B) TYPE: amino acid
(C) STRANDEDNESS:
(D) TOPOLOGY: linear

(ii) MOLECULE TYPE: peptide

(v) FRAGMENT TYPE: internal

(xi) SEQUENCE DESCRIPTION: SEQ ID NO: 4:

Phe Ile Ser Glu Ala Ile Ile His Val Leu His Ser Arg Ser Ser Ala
1               5                   10                  15

Arg Ser Arg Ile Glu Asp
            20

## Description

### Field of the Invention

This invention relates to a method of preparing antibodies, a method of detecting *Salmonella* bacteria, monoclonal antibodies substantially specific for *Salmonella* bacteria, and to cell lines, hybridomas etc. producing the monoclonal antibodies.

### Background of the Invention

*Salmonella* bacteria generally are motile in liquid or semi-solid media by means of peritrichous flagella. These organelles are powered by a basal body, which acts as an intracellular motor and transmits torque through the bacterial cell wall to an extracellular structure, the filament. When the organism swims, filaments asociate into twisted bundles whose motion drives the cell through the medium. The filaments are extremely long, helical structures, extending 10 µm or more from the cell surface, and consist of a single self-polymerising protein, flagellin. Flagellar filaments grow by addition of new flagellin monomers to the distal tip, which they reach after travelling along the hollow central channel of the filament; this channel extends into the cell through so-called hook and rod structures which are co-axial with the filament.

*Salmonella* flagellins carry the H antigen, one of the major antigens that elicit an immune response in an infected animal. In addition to being strongly antigenic, H antigens are also highly diverse, both in length and in sequence. The amino acid sequence of the antigenic region of the flagellin molecule is highly polymorphic: over 50 flagellins can be distinguished by serology. In fact, the diversity of the H antigens, together with that of the somatic lipopolysaccharide (O) antigens, forms the basis of the official (Kauffmann-White) scheme for the classification of Salmonellae. This scheme identifies serovars of *Salmonella* by a unique antigenic formula of O and H antigens. Currently there are over 2300 recognised serovars of *Salmonella.*

*Salmonella*-mediated food poisoning is a major problem in many countries. Accordingly, there is a need for tests in the food industry, and in the medical and veterinary fields, which are capable of reliably detecting the presence of *Salmonellae.* Such tests are needed to ensure that foodstuffs and animal feedstuffs are safe for consumption, to aid diagnosis for clinicians and veterinarians, and to assist epidemiologists in tracking outbreaks of disease.

Currently available tests fall into two general categories: biological or immunological. Biological tests require culture of samples for a prolonged period, detection of *Salmonella* organisms being based on biochemical and/or growth characteristics of the bacteria. Accordingly, such tests take 3-5 days to produce results and are not rapid enough for many purposes. Immunological tests are based on the serological or antigenic characteristics of the bacteria. Whilst such immunological tests may require some culture of samples, the duration of culture is far shorter than in biological tests, such that immunological tests may generally be performed more rapidly than biological tests.

Originally, immunological tests relied on the use of polyclonal antisera (reviewed by Thomason, 1980 J. Food Sci. 44, 381), but these were less than ideal in that they were insufficiently specific for *Salmonella*, giving rise to many false positive results. Subsequently, various monoclonal antibodies were raised against *Salmonellae* (Mattingly *et al*., 1985 Food Technology 90-94; Tsang et *al*., 1987 Infection and Immunity 55, 211-216; Paterson & Tiffin, 1988 Letts. in Applied Microbiol. 7, 41-43; and Todd *et al.,* 1991 Food Microbiol. 8, 311-324). Monoclonal antibodies have several advantages over polyclonal sera: they are generally more specific and available at consistent quality.

It is desirable that any single antibody should bind to all strains of *Salmonella*, whilst ideally not binding to any organism which is not a *Salmonella* species. In laboratory conditions, testing deliberately contaminated samples, the best test devices currently available (using ELISA-based assays) perform very well but in practical conditions, using real food samples, their performance is less satisfactory, often giving rise to false positive results in the presence of one or more of several non-*Salmonella* organisms. In addition the anti-*Salmonella* antibodies, upon which conventional ELISAs are based, fail to bind to several *Salmonella* strains, such that a number of monoclonal antibodies, of different specificities, are required in combination for best results.

There is thus an unmet need for an improved immunoglobulin which, ideally, in tests will detect all or virtually all *Salmonella* organisms whilst giving a lower rate of false positive results than those antibodies currently available.

One of the objects of the present invention is to provide such an immunoglobulin.

### Summary of the Invention

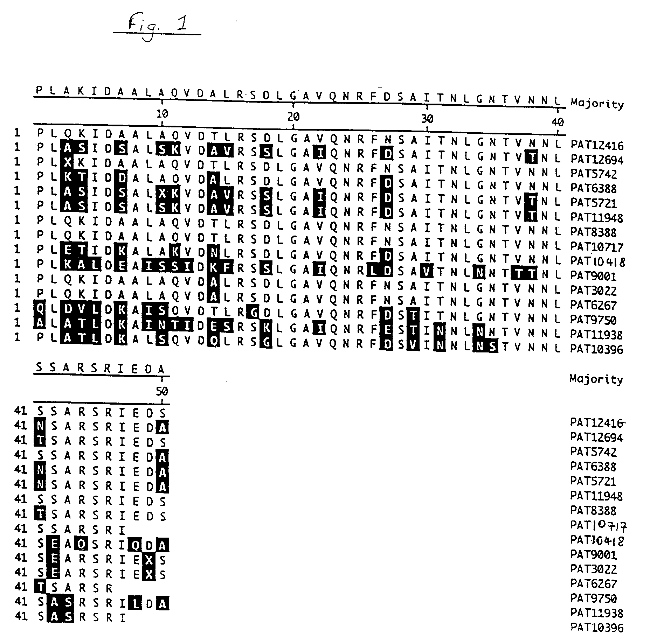

In a first aspect, the invention provides an immunoglobulin molecule or an antigen-binding variant thereof which binds to an epitope present within amino acid residues 403 to 452 (Seq ID No. 1 in the attached sequence listing) of the flagellin protein of *Salmonella typhimurium* PIR accession No. A24262, or the equivalent portion from other *Salmonella* strains. (Flagellins from different strains vary in length, so the amino acid residue numbering is not universal. However sequence comparisons, with the benefit of the present disclosure [e.g. Figure 1], will enable the person skilled in the art readily to identify such equivalent portions).

The immunoglobulin molecule is typically an antibody (most preferably a monoclonal antibody), which may be of any class and sub-type, but is typically IgG. The term "antigen-binding variant" refers to molecules which comprise the antigen-binding portion of an immunoglobulin (which antigen-binding is conferred primarily by the complementarity determining regions, or CDRs), whether obtained by traditional immunisation techniques, or obtained by genetic manipulation of appropriate coding sequences, or by *in vitro* screening of (e.g. phage) libraries expressing antibody-like molecules.

Examples of molecules falling within the meaning of the term "antigen-binding variant" of an immunoglobulin include, but are not limited to, the following:
Fv antibody fragments (Skerra & Pluckthun, 1988 Science **240,** 1038);
ScFv antibody fragments (Bird *et al,* 1988 Science **242,** 423);
bi-specific antibody fragments where one or both binding specificities is in accordance with the invention defined above (Holliger *et al,* 1993 Proc. Natl. Acad. Sci. USA **90,** 6444);
bi-specific antibody fragments where one binding specificity is in accordance with the invention defined above, and the other binding specificity is for a marker molecule, reagent or the like (e.g. an enzyme);
antibody fragments obtainable from, derived from, or encoded by nucleic acid sequences obtainable from *Camelidae* (WO 94/04678, WO 94/25591); and
antibody-type molecules isolated from, or encoded by nucleic acid sequences obtainable from, synthetic biomolecular libraries (e.g. Lerner *et al,* 1992 Science **258,** 1313).

In the following text, references to "immunoglobulin" should be construed as including reference to antigen-binding variants thereof, where the context so permits.

It will be apparent from the foregoing that the immunoglobulin or antigen-binding variant thereof may be a naturally occurring molecule (e.g. antibody) obtained by traditional immunisation techniques, or may be obtained or altered by recombinant DNA techniques so as to comprise a synthetic portion or be wholly synthetic. Such partially or wholly synthetic molecules may be engineered, for example, to alter affinity, or number of binding sites, or number of binding specificities.

The immunoglobulin or antigen-binding variant thereof in accordance with the invention may be in isolation from other immunoglobulin molecules (or variants thereof) directed against other epitopes. More especially, the molecules of the invention may preferably be in isolation from immunoglobulin molecules (or variants thereof) directed against portions of *Salmonella* flagellin other than residues 403-452 or directed against other *Salmonella* antigens (e.g. "O" antigens of LPS).

The present inventors have found that this portion (i.e. equivalent to residues 403-452 of *S. typhimunium* flagellin) of the flagellin protein is well conserved amongst bacteria of the genus *Salmonella*, whilst being rather different in sequence to the flagellin proteins of other bacterial genera. The inventors have found that antibodies directed against epitopes present in this portion of flagellin protein will tend to bind to all or substantially all strains of *Salmonella*, whilst binding to very few non-*Salmonella* bacteria, even those closely-related, such as Escherichia, Klebsiella, Citrobacter etc.

Desirably the immunoglobulin molecule (or antigen-binding variant thereof) in accordance with the invention binds to an epitope present within amino acid residues 418 to 452 of the *Salmonella typhimurium* flagellin protein, which residues tend to be more highly conserved than residues 403-417. More preferably, the immunoglobulin will bind to an epitope present within amino acid residues 427 to 436, or to an epitope present within amino acid residues 444 to 451, of the *Salmonella typhimurium* flagellin protein, which portions are especially well-conserved among *Salmonellae.*

This sequence of residues 427 to 436 in *Salmonella* is generally RFDSAITNLG (Seq ID No. 2 in the attached sequence listing), although certain strains have been found to possess a slight variant RF**N**SAITNLG. Immunoglobulins (or antigen-binding variants thereof) which bind to either one, or both, of these variants may be of use in the present invention. The sequence of residues 444 to 451 is generally SARSRIED (Seq ID No. 3 in the attached sequence listing).

A preferred immunoglobulin in accordance with the present invention is a monoclonal antibody obtainable from a hybridoma designated 5836.1, which antibody binds to an epitope present in residues 427-436 and has been found to detect all motile strains of *Salmonella* (i.e. those with flagella) so far tested. Another preferred immunoglobulin is a monoclonal antibody obtainable from a hybridoma designated 6035.1, which antibody binds to an epitope present in residues 444 to 451.

The immunoglobulin molecule (or antigen-binding variant) of the invention may be provided as a serum sample, or in the form of a crude culture supernatant, or ascites fluid, or cells producing the immunoglobulin, or may be purified (e.g. by affinity chromatography and the like). Further, the antibody may be frozen, lyophilised, immobilised on a solid support etc. all of which may be performed by methods well-known to those skilled in the art.
The invention also provides a host cell capable of producing an immunoglobulin molecule or antigen-binding variant thereof which binds to an epitope present within amino acid residues 403 to 452 of the flagellin protein of *Salmonella typhimurium* PIR accession No. A24262, or the equivalent portion of flagellin from other *Salmonella* strains. The host cell may be prokaryotic (e.g. bacterial), such as a bacterium harbouring a phage encoding the immunoglobulin-like molecule. Typically, however, the host cell is eukaryotic, and preferably mammalian. Desirably the host cell is an immortalised cell line, such as a hybridoma (e.g. hybridoma 5836.1 or 6035.1, mentioned above), and produces a monoclonal antibody molecule.

The invention further provides a nucleic acid sequence encoding an immunoglobulin molecule or antigen-binding variant thereof, which binds to an epitope present within amino acid residues 403 to 452 of the flagellin protein of *Salmonella typhimurium* PIR accession No. A24262, or the equivalent portion of flagellin from other *Salmonella* strains. Such a nucleic acid may be obtainable, for example, from hybridoma 5836.1 or 6035.1, by standard nucleic acid isolation methods known to those skilled in the art (e.g. from a phage library).

In a further aspect the invention provides a method of producing an immunoglobulin molecule or antigen-binding variant thereof, the method comprising immunising a mammal with an immunogen which comprises at least one B-cell epitope present within amino acid residues 403 to 452 of the flagellin protein of *Salmonella typhimurium* PIR accession No. A24262, or the equivalent portion of flagellin from other *Salmonella* strains, said B-cell epitope being in substantial isolation from *Salmonella* flagellin residues outside the portion 403 to 452. Conveniently the immunogen is a synthetic peptide and preferably several immunisations are performed, typically using adjuvants such as Freund's Complete or Incomplete Adjuvant. Methods of preparing hybridomas, and hence monoclonal antibodies, from suitably immunised animals are well known (e.g. Kohler & Milstein, 1975 Nature 256, 495; Newell *et al.,* 1988 "Making Monoclonals" PHLS, London pp1-94). Preferably the immunogen will comprise the flagellin epitope linked or fused to an amino acid sequence comprising one or more T helper cell epitopes, so as to facilitate induction of an antibody response. Such T helper cell epitopes may conveniently be provided by fusion of the flagellin epitope to a carrier protein (such as bovine serum albumin, or keyhole limpet haemocyanin), or chemical cross-linking to a protein. Suitable T helper cell epitopes are well known to those skilled in the art, including epitopes present in sperm whale myoglobin.

In an additional aspect, the invention provides a method of producing an immunoglobulin molecule or antigen-binding variant thereof, the method comprising the steps of: obtaining a library of nucleic acid sequences encoding immunoglobulin molecules or antigen-binding variants thereof; screening the library with a screening molecule which comprises at least a portion of amino acid residues 403 to 452 of the flagellin protein of *Salmonella typhimurium* PIR accession No. A 24262, or the equivalent portion of flagellin from other *Salmonella* strains, said portion being in substantial isolation from *Salmonella* flagellin residues outside the portion 403 to 452; selecting those nucleic acid sequences which encode an immunoglobulin or antigen-binding variant which binds to the screening molecule; and causing expression of the selected nucleic acid sequences to obtain the desired immunoglobulin or antigen-binding variant thereof.

Conveniently the screening molecule comprises a synthetic peptide which may optionally be conjugated to, or fused with, a carrier molecule. Appropriate libraries of nucleic acid sequences in a form suitable for screening are well-known to those skilled in the art (e.g. Lerner *et al,* 1992 Science **258,** 1313; McCafferty *et al,* 1990 Nature **348,** 552).

If desired, the selected nucleic acid sequences may be further screened (e.g. with screening molecules which comprise portions of flagellin from non-*Salmonella* species), to identify immunoglobulin molecules or antigen-binding variants thereof which have optimum specificity for *Salmonella* flagellin epitopes (i.e. minimum cross-reactivity for non-*Salmonella* species) but which react with as many strains of *Salmonella* as possible. (Similar screening will desirably be applied to immunoglobulins obtained by conventional immunisation techniques.)

In a further aspect, the invention provides a method for detecting the presence of *Salmonella* organisms, comprising contacting a sample to be tested with an immunoglobulin molecule or antigen-binding variant thereof which binds to an epitope present within amino acid residues 403 to 452 of the flagellin protein of *Salmonella typhimurium* PIR accession No. A24262, or the equivalent portion of flagellin from other *Salmonella* strains; and monitoring binding of the immunoglobulin molecule, or antigen-binding portion thereof, to the sample. Desirably the immunoglobulin molecule is an antibody (e.g. monoclonal antibody) having the preferred characteristics defined above.
It will generally be desired for the sample to be subjected to a processing step prior to contact with the immunoglobulin molecule or antigen-binding variant, the purpose of which is to ensure that the epitopes of the flagellin protein of any *Salmonella* bacteria present in the sample are exposed and available for binding to flagellin-specific immunoglobulins. Conveniently this is accomplished by boiling of the sample for a short period, or otherwise by processing the sample with denaturing agents such as surfactants and the like.

Those skilled in the art will appreciate that numerous assay formats can be envisaged for performing the method defined above. Preferred embodiments include enzyme-linked immunosorbent assay (ELISA), immunochromatographic assays, or Western blots. Generally, the assay method will normally involve binding of the immunoglobulin molecule (or antigen-binding variant thereof) of the invention to *Salmonella* flagellin, if present in the sample. The immunoglobulin molecule may be labelled to facilitate detection of such binding. A variety of labels are known to those skilled in the art and include radioactive labels, enzyme labels, coloured latex particles, and biochemical labels (such as biotin and the like). Alternatively, binding of the immunoglobulin molecule to *Salmonella* flagellin may be detected by use of a second immunoglobulin, which immunoglobulin may be labelled and will preferably also be a monoclonal antibody.

A large number of antibody-based *Salmonella*-detection methods have been described in the prior art (e.g. Van Poucke, 1990 Appl. Environ. Microbiol. 56, 924-927; Anon., 1987 J. Assoc. Off. Anal. Chem. 70, 396-397; Cerqueira-Campos *et al.,* 1986 Appl. Environ. Microbiol. 52, 124-127; Ibrahim *et al.,* 1986 J. Food Protect. 49, 92-98) and, in principle, any of these could be adapted for use with an immunoglobulin molecule or variant thereof in accordance with the invention. Further details of specific envisaged assay methods are given in the examples below.

In a further aspect, the invention provides an assay device for use in detecting the presence of *Salmonella* organisms, the assay device comprising an immunoglobulin molecule or antigen-binding variant thereof in accordance with the invention. Again, in principle, any of the assay devices known in the prior art (e.g. D'Aoust & Sewell, 1988 J. Food Protect. 51, 538-541; Farber *et al.,* 1985 J. Food Protect. 48, 790-793) could be adapted for use with an immunoglobulin molecule in accordance with the invention. Desirably the device will comprise a solid support (e.g. microtitre plate, glass slide, latex bead, chromatographic test stick or the like) provided with an immunoglobulin molecule (or antigen-binding variant thereof) in accordance with the invention.

A preferred embodiment of the assay device is an immunochromatographic device, such as the Clearview™ device sold by Unipath Limited. Such devices are easy to use and are reliable. In particular, the device may comprise two or more immunoglobulin molecules (or antigen-binding variants thereof), each molecule binding a respective epitope present in Salmonella, and in particular, each molecule binding a respective epitope present in the region of 403 to 452 of the *Salmonella typhimurium* flagellin. Conveniently, one such immunoglobulin molecule will be directed against an epitope present in residues 427-436, and another such immunoglobulin molecule will be directed against an epitope present in residues 444-451. Embodiments of this type facilitate use of a "sandwich" type assay device and method, in which *Salmonella* organisms are captured on a solid support with a first immunoglobulin, and detected by binding to a second (typically) labelled immunoglobulin.

The assay method and assay device of the invention are, in preferred embodiments, able to detect the majority (over 85%, preferably over 95%) of pathogenically important *Salmonella* species (i.e. those which generally cause infections in man), whilst giving a very low (less than 2%) false positive rate. The assay method and device may particularly be used for detecting the presence of *Salmonella* in foods and/or in food preparation areas.

The present inventors consider that the amino acid residues 403 to 452 of the flagellin protein have unexpectedly useful properties (e.g. are useful in preparing monoclonal antibodies in accordance with the invention). Accordingly, in another aspect the invention provides a peptide comprising at least part of amino acid residues 403 to 452 of the flagellin protein of *S*. *typhimurium* PIR accession No. A24262, or the equivalent portion from other *Salmonella* strains, in substantial isolation from other portions of the flagellin sequence. The peptide is preferably in antigenic and/or immunogenic form and preferably substantially pure. The peptide preferably comprises at least amino acid residues 427 to 436, and/or at least residues 444 to 451, of the flagellin sequence. The peptide may desirably be fused or chemically coupled to other molecules (such as polypeptides or peptides which comprise T helper cell epitopes).

In addition to raising antibodies, the particular peptide sequence of the flagellin protein identified by the inventors may be useful as a reagent (e.g. included as a component in an assay method and/or an assay kit for detecting the presence of *Salmonella* organisms). When used as a reagent in an assay, the peptide may desirably be joined to a labelling and/or anchoring moiety (e.g. biotin, or avidin) to label the peptide and/or facilitate immobilisation of the peptide to a solid surface.

Accordingly in another aspect the invention provides for a method of detecting the presence of *Salmonella* organisms, comprising use of a peptide comprising at least part of amino acid residues 403 to 452 of the flagellin protein of *S. typhimurium* PIR accession No. A24262, or the equivalent portion from other *Salmonella* strains, in isolation from other portions of the flagellin sequence. The invention also provides for an assay device for detecting the presence of *Salmonella* organisms, the device comprising the peptide defined above.

In one embodiment, the peptide of the invention is used in an assay method and/or device involving displacement. For example, immobilised peptide could be displaced by the introduction of a sample comprising *Salmonella* organisms. Preferably the peptide would be provided with a label (e.g. enzyme, or coloured bead) to facilitate detection of the displacement of the peptide. Displacement assays of this type are described, for example, in WO 91/05262 and PCT/EP95/04518.

The invention will be further described by way of illustrative example and with reference to the accompanying drawing, Figure 1, which is a comparison of the sequence of amino acid residues 403 to 452 of the flagellin protein of *S. typhimurium* A24262 with the homologous sequences of various bacteria, including other *Salmonella* strains, and *E. coli* and *Citrobacter* strains.

### Example 1

The inventors and their colleagues first used a *Salmonella* whole cell extract or a *Salmonella* flagellin preparation to make monoclonal antibodies against *Salmonella* "O" antigen (lipopolysaccharide, LPS) or "H" antigen (flagellin) respectively. The binding sites of the various monoclonal antibodies raised against H antigens were roughly mapped by using them to probe Western blots of trypsin-digested flagellin.

The inventors observed that antibodies which bound to the central portion of the 490 amino acid flagellin protein tended to be too specific for use in an assay, in that whilst they did not bind to the flagellin protein of non-*Salmonella* organisms (and so would not give false positive results) they equally would not bind to the flagellin of every *Salmonella* strain, such that some strains might go undetected in a test. (A similar observation has been reported by van Asten *et al.,* 1995 J. Bacteriol. 177, 1610-1613).

Conversely, antibodies which bound at the N- or C-terminal regions tended to be insufficiently specific, in that whilst they bound to all *Salmonella* strains, they also bound to large numbers of non-*Salmonella* organisms.

The inventors hypothesised that antibodies which bound to the regions between the central portion and the N- or C-terminal may combine the desired characteristics of high specificity for *Salmonella*, whilst still binding to all *Salmonella* strains. Accordingly the inventors set out to prepare such monoclonal antibodies.

A search of various computer databases was conducted to find reported flagellin sequences. The search included protein translations of coding sequences on the GenBank database (National Institutes of Health, MD, USA), the SWISS-PROT database maintained by the University of Geneva and the European Molecular Biology Laboratory (EMBL) data library, and the Protein Information Resource (PIR) Protein sequence database (National Biomedical Research Foundation, Washington D.C.). Flagellin protein sequences recovered from the databases were aligned by the Clustal method using the Megalign facility on DNA-Star software. Sequences available for comparison were limited to a few strains of *Salmonella, E. coli*, *Proteus, Serratia, Yersinia, Pseindomonas* and *Campylobacter*. Of particular interest were the flagellins of Enterobacteriaceae which typically cause false positives in *Salmonella* immunoassays: *Citrobacter, E. coli* and *Proteus.*

From this comparison, a stretch of 50 amino acids, corresponding to residues 403 to 452 of the *S. typhimurium* sequence PIR accession A24262 was selected as being relatively well conserved among the *Salmonellae* while exhibiting some differences between *Salmonellae* and non-*Salmonellae*. Flagellins from different strains vary in length, so the amino acid numbering is not universal.

A few *Salmonella flagellin* sequences were already known, but the inventors determined several more. Figure 1 shows a comparison of the amino acid sequence in this 50 residue region (403-452) for various strains of *Salmonella* and some non-*Salmonella* bacteria. The consensus sequence ("majority") is shown above and generally corresponds to that of *S. typhimurium* A24262 flagellin. The numbers to the right of the sequence refer to the culture collection numbers of the various strains examined and correlate with those shown in Table 1.

Within this fifty amino acid region, the ten amino acid sequence RFNSAITNLG, corresponding to residues 427 to 436 of the *S. typhimurium* A24262 sequence, was chosen as being especially specific to the *Salmonellae.* This sequence was therefore selected for synthesis as peptide immunogen. Since many *Salmonella* serotypes have the sequence variant RFDSAITNLG it was necessary to select for antibodies which were indifferent to this variation. This was achieved by screening the hybridomas for their ability to bind to flagellins of the two types (represented here by *S. typhimurium* and *S. tennessee* "6388").

The inventors subsequently determined the sequences of the flagellins of a number of additional *Salmonellae* and non-*Salmonellae,* most significantly the *Citrobacter* strains and an unusual strain of *E. coli* (NCTC 10418). Certain *Citrobacter* strains and *E. coli* NCTC 10418 contain the peptide RFNSAITNLG in its entirety and therefore would be expected to bind to any *Salmonella*-binding MAb raised against that peptide sequence.

### Synthesis of Peptide

An RFNSAITNLG "Multi-Antigenic Peptide" (MAP) was produced on the NovaSyn Gem™ semi-automatic solid phase peptide synthesiser. The solid support was Kieselguhr [NovaSyn KA 100] derivatised with a branching octavalent lysine core with the following structure:
Fmoc₈₋Lys₄₋Lys-₂₋Lys- -Ala-Solid support

The following peptide sequence was built up on the support using standard Fmoc chemistry:
(Lys-Lys-Arg-Phe-Asn-Ser-Ala-Ile-Thr-Asn-Leu-Gly)₈- [lysine core]- -Ala-Solid support

The peptide comprises the residues 427 to 436 of the *S. typhimurium* sequence, preceded by two Lys residues which were included to improve the water-solubility of the peptide and are not part of the flagellin sequence. Following synthesis, the peptide was cleaved from the support using trifluoroacetic acid/tri-isopropyl silane solution and concentrated by evaporation. The peptide was extracted three times with diethyl ether and dried under vacuum. The peptide was dissolved in distilled H₂O and dialysed overnight against distilled H₂O. The peptide was freeze-dried and stored at -20 C. A sample of the MAP was analysed on an Applied Biosystems automated protein sequencer. This confirmed that the sequence had been authentically synthesized as above.

### Immunisation with Peptide

A BALB/C mouse initially received, by the subcutaneous and intraperitoneal routes, a total of 50 g MAP emulsified in Freund's Complete Adjuvant. Two months later it received, by the subcutaneous and intraperitoneal routes, a total of 50 g MAP emulsified in Freund's Incomplete Adjuvant. Immunisation in Freund's Incomplete Adjuvant was repeated after 2.5 weeks. A first test tail bleed followed after 1 week. The mouse was immunised with a further 50 g of MAP by the intraperitoneal route ten days later. A second test tail bleed followed after two weeks. One month later the mouse was immunised with 50 g of purified flagellin from *S. typhimurium* and 50 g of purified flagellin from *S. tennessee*. A third test tail bleed was performed nine days later. After seven weeks the mouse received 100 g MAP intravenously. Three days later the animal was sacrificed and its spleen removed for preparation of hybridomas.

### Tail Bleed Testing

The antigens used for testing by ELISA were MAP, prepared as above, and flagellins purified from plate cultures of *S. typhimurium* and *S. tennessee* by a differential centrifugation protocol modified from that of Kerridge *et al*. [1962 J. Mol. Biol. **4** pp 227-238]. Flagellin preparations were boiled for 5 minutes prior to dilution in carbonate buffer to improve the exposure of *Salmonella*-specific epitopes. Greiner high-capacity binding microtitre plates were sensitised overnight at 4 C with 100 l of antigen (MAP diluted to 5 g/ml, or flagellins diluted to 1 g/ml in 0.05 M sodium carbonate buffer, pH 9.6). The wells were washed with PBS and then blocked with 200 l of PBS containing 0.05% Tween-20 and 1% bovine serum albumin ("PBST/BSA") at 37 C for 1 hour. The wells were then washed in PBS. 100 l samples of tail bleed sera, in serial 10-fold or 5-fold dilutions made in PBST, from immunised and non-immunised mice, were added to the wells for 2 hours at 37 C. The wells were again washed with PBS. 100 l of rabbit anti-mouse IgG/IgA/IgM-alkaline phosphatase conjugate [Zymed] diluted 1/1000 with PBST/BSA was added for 1 hour at 37 C. The wells were then thoroughly washed with PBS and 100 1 of *p*-nitrophenyl phosphate [Sigma 104 Phosphatase Substrate tablets, dissolved at 1mg/ml in 1 M diethanolamine, pH 9.8] was added for 30-60 minutes at 37 C. Yellow colour development was measured using a spectrophotometer at 410 nm.

Wells coated with MAP gave signals above background levels with the first tail bleed serum from the immunised mouse at a dilution of 1/100,000; whereas wells coated with flagellins reacted at 1/100 dilution of serum. With the second tail bleed, the flagellins reacted with serum at 1/1000 dilution. With the third tail bleed, *S. typhimurium* flagellin reacted at 1/12500 dilution of serum and *S. tennessee* flagellin at 1/62500.

### Preparation of Hybridomas

The spleen removed from the immunised mouse was perfused with RPMI medium to collect spleen cells. Approximately 5 x 10⁷ spleen cells were fused with 4 x 10⁷ SP2 mouse myeloma cells in the presence of polyethylene glycol by standard methods (Kohler & Milstein, 1975 Nature **256,** 495). Fused cells were diluted in selective medium (RPMI containing 10% horse serum, 2.4 mM L-Glutamine, 1.2 mM sodium pyruvate, 60 I.U./ml penicillin, 60 g/ml streptomycin, 10⁻⁴ M hypoxanthine and 1 g/ml azaserine) and plated out in twelve 48-well culture plates. The plates were incubated at 37 C in 5% CO₂ for one week. The selective medium was then replaced by growth medium (RPMI containing 5% horse serum, 2.4 mM L-Glutamine, 1.2 mM sodium pyruvate, 60 I.U./ml penicillin, and 60 g/ml streptomycin). Two days later the hybridoma supernatants were screened by ELISA for binding to MAP and flagellins, as detailed below.

Greiner high-capacity binding plates were sensitised with MAP or boiled flagellin from *S. typhimurium*, washed, and blocked, as described for Tail Bleed Testing above. 100 l of individual hybridoma well supernatant was added to the wells for 2 hours at 37 C. Following washing with PBS, the bound antibody was detected with conjugate and colorimetric substrate as described for Tail Bleed Testing. Colour development at 37 C was measured after 1 hour. Of 576 hybridoma wells screened, 38 produced antibodies which reacted with both MAP- and flagellin-sensitised wells. After expansion, these hybridomas were retested in ELISA for binding of supernatant antibody to 4 different immobilised antigens: MAP; boiled flagellin from *S. typhimurium;* boiled flagellin from *S. tennessee;* and bovine serum albumin. The microtitre plate wells were sensitised overnight at 4 C with antigens at 1 g/ml in carbonate buffer, except MAP which was at 5 g/ml. Assay steps were as described above. One hybridoma was found to bind strongly to MAP and the two flagellins, but not to BSA, and was therefore selected for further analysis.

### Subcloning

The MAP- and flagellin-binding hybridoma was subcloned by the method of limiting dilution. Subclones were re-tested for their ability to bind specifically to MAP and the two flagellin types. The hybridoma was isotyped after subcloning using isotype-specific rabbit anti-mouse-alkaline phosphatase conjugates and found to be of the IgG1 class.

### Specificity testing

Overnight cultures of bacterial strains (17 motile strains of *Salmonella* plus 19 other *Enterobacteriaceae*) in brain/heart infusion (BHI) broth were boiled for 30 min and centrifuged for 5 minutes at 13,000 rpm in a microcentrifuge. The culture supernatants (which contain shed flagella) were diluted 1:10 with carbonate buffer and 100µl used to sensitise the wells of Greiner high capacity binding plates overnight at 4 C. Hybridoma supernatants were tested for binding to the wells by ELISA as described above. The subclone showing the most uniform reaction with the *Salmonellae* and the least reaction with the other *Enterobacteriaceae* was selected for re-cloning. Following re-cloning, the specificity of the hybridoma was re-confirmed using the same bacterial panel. The re-cloned hybridoma cell line was designated 5836.1.

### Purification of MAb 5836.1

A culture supernatant of cell line 5836.1 in serum-free medium was filtered to remove cells and loaded onto a PROSEP^{R} High Capacity Protein A column [bioProcessing Inc.] in 1M borate buffer pH 8.5 containing 0.15M NaCl. Following washing in binding buffer, the MAb was eluted in 0.1M citrate buffer, pH 3.0. Purified MAb 5836.1 was tested for binding in ELISA to immobilised crude supernatant antigens (prepared as above) from *Salmonella* and *Enterobacteriaceae* strains. The panel comprised:
1. *Salmonella* serotypes common in food outbreaks (according to UK Public Health Laboratory Service figures): *S. enteritidis, S. typhimurium,* and *S. virchow.*
2. *Salmonella* serotyopes which include strains known not to bind to some earlier "*Salmonella*-specific" MAbs: *S. derby, S. tennessee, S. ealing, S. muenchen,* and *S. rubislaw.*
3. Type strains of *Enterobacteriaceae* (potential cross-reactors).
4. An unusual strain of *E. coli* (NCTC 10418) which the inventors' studies have shown to contain sequence identities with *Salmonella* in the region of the flagellin MAP peptide.

The results are shown in Table 1. Monoclonal antibody from the 5836.1 hybridoma is able to detect all motile strains of *Salmonella* so far tested. It cross-reacts with *E. coli* NCTC 10418 and, to a certain extent, with certain *Citrobacter* strains which are now known to have flagella possessing the *Salmonella* sequence RFNSAITNLG.

**Table 1**

| **Strain** | **Detected by 5836.1** | **Comments** |
|---|---|---|
| S. Typhimurium NCTC 12416 | YES | Type Strain |
| S. Enteritidis NCTC 12694 | YES | Type Strain |
| S. Virchow NCTC 5742 | YES | |
| S. Tennessee NCTC 6388 | YES | |
| S. Derby NCTC 5721 | YES | |
| S. Ealing NCTC 11948 | YES | |
| S. Muenchen ATCC 8388 | YES | |
| S. Rubislaw ATCC 10717 | YES | |
| *Escherichia coli* NCTC 10418 | YES | Flagellin has "Salmonella" epitopes |
| *E. coli* NCTC 9001 | YES [Weak] | |
| *E. coli* NCTC 11954 | NO | |
| *Citrobacter diversus* | NO | |
| *C. feundii* NCTC 6267 | YES [Very weak] | |
| *C. freundii* NCTC 9750 | NO | Type Strain |
| *C. koseri* NcTc 10786 | NO | Type Strain |
| *Proteus mirabilis* NCTC 11938 | NO | Type Strain |
| *Klebsiella sp.* | NO | |
| *Enterobacter* NCTC 10005 | NO | Type Strain |
| *Edwardsiella tarda* NCTC 10396 | NO | Type Strain |

### Example 2

A second peptide was prepared, using conventional peptide synthesising chemistry, which comprised the sequence SARSRIED, corresponding to amino acid residues 444-451 of the flagellin protein of *S. typhimurium* strain A24262, fused at its N terminal to a T cell epitope from sperm whale myoglobin (which is known to be well-recognised by the MHC Class II molecules of BALB/c mice - Prieto *et al,* 1995 Eur. J. Immunol. **25**, 877-885). The sequence of the resulting peptide, known as T₂-SARS, is shown below:
(Seq ID No. 4 in the attached sequence listing)
   FLSEAIIHVLHSRS**SARSRIED**

### Preparation of anti-T₂-SARS hybridomas

A set of four BALB/c mice were immunised with the T₂-SARS peptide essentially as described in Example 1 above.

The three responding mice from the set were sacrificed and their spleen cells used to create hybridomas. The initial screenings (using as solid phase antigens *S. typhimurium* flagellin, or T₂-SARS peptide and BSA) indicated that of 1440 clones tested, five reacted with both flagellin and T₂-SARS peptide, while six reacted with T₂-SARS peptide alone. In addition, and rather surprisingly, many clones were found which reacted with flagellin alone; most of these originated from the mouse which had shown the strongest anti-flagellin serum titres.

The most promising hybridomas were subcloned and tested in ELISA for binding to crude supernatant flagellin antigens of *Salmonella* and cross-reactor strains. Only one hybridoma, 6035.1, showed a satisfactory degree of specificity for *Salmonella,* with cross-reactivity to *E.* *coli* NCTC 10418 and *Citrobacter* strain CMCC 3389. There was very little reaction of 6035.1 with *Citrobacter* strains NCTC 6267 and CMCC 3022, (i.e. those containing the epitope EARSRIED rather than SARSRIED). Thus the object of the immunisations was accomplished and a monoclonal antibody identified which could distinguish *Citrobacter* strains on the basis of a single amino acid alteration.

The results of the ELISA tests (conducted as described in Example 1) are shown in Table 2, which details the results obtained with the monoclonal antibodies from hybridoma 6035.1 (described above), 5836.1 (described in Example 1), and a further hybridoma obtained by the inventors (5972.1), which are compared with two monoclonal antibodies produced by prior art hybridomas (3034.13 and 3020.13).

**Table 2**

| **Strain** | ELISA Result | | | | |
|---|---|---|---|---|---|
| | 3034.13 | 3020.13 | 5836.1 | 5972.1 | 6035.1 |
| *S. Typhimurium* NCTC 12416 [T] | + | + | + | + | + |
| *S. Enteritidis* NCTC 12694 [T] | + | + | + | + | + |
| *S. Virchow* NCTC 5742 | + | + | + | + | + |
| *S. Tennessee* NCTC 6388 | + | - | + | + | + |
| *S. Derby* NCTC 5721 | + | - | + | + | + |
| *S. Ealing* CMCC 2071 | + | - | + | + | + |
| *S. Ealing* NCTC 11948 | + | + | + | + | + |
| *S. Muenchen* ATCC 8388 | + | - | + | + | + |
| *S. Rubislaw* ATCC 10717 | + | + | + | + | + |
| *E. coli* NCTC 10418 | + | - | + | + | + |
| *E. coli* NCTC 9001 | - | - | + | - | - |
| *Citrobacter diversins* CMCC 3022 | + | + | - | + | - |
| *Citrobacter koseri NCTC10786[T]* | + | - | - | - | - |
| *Citrobacter freundii* NCTC 6267 | + | + | + | + | - |
| *Citrobafter freundii CMCC3389* | + | - | + | + | + |
| *Citrobacter freundii* NCTC9750[T] | + | - | - | + | - |
| *Proteus mirabilis NCTC* | + | - | - | - | - |
| *11938[T]* | | | | | |
| *Klebsiella sp. CMCC 2703* | *-* | *-* | *-* | *-* | *-* |
| *Enterobacter NCTC 10005 [T]* | + | - | - | - | - |
| *Edwardsiella tarda NCTC 10396* | + | - | - | - | - |

| | | | | | |
|---|---|---|---|---|---|
| **+ = positive ELISA result** | | | | | |
| **- = negative ELISA result** | | | | | |
| **(Strains with the suffix CMCC are proprietary to the applicant)** | | | | | |

### Example 3

Immunoglobulins in accordance with the invention, as exemplified by the monoclonal antibody obtainable from hybridoma 5836.1 or 6035.1, could be useful in any of a large number of different assay formats for detection of *Salmonella*. Such assays include ELISA, immunochromatographic and Western blot-type assays. Details of such assays are proposed below.

In one embodiment, flagella antigen present in a sample to be tested could be deposited on a solid phase (e.g. microtitre well surface) and used to capture *Salmonella* flagellin-specific immunoglobulin from solution. Bound immunoglobulin could be detected using, for example, an anti-mouse antibody/enzyme conjugate. Alternatively, the immunoglobulin itself could be used as a conjugate with the enzyme. Methods for preparing such conjugates are well-known to those skilled in the art.

In an alternative embodiment, flagellin-specific immunoglobulin (e.g. a monoclonal or MAb) could be attached to a solid phase and used to capture flagella antigen from bacterial culture. Antigen would be detected by a labelled second antibody (e.g. conjugated to enzyme). The second antibody would be capable of binding all the *Salmonella* strains bound by the first antibody/MAb and might additionally bind some Enterobacterial strains not bound by the first antibody. The overall specificity, however, would remain that of the MAb. Alternatively, the second antibody could be bound to the solid phase and used to capture antigen and the MAb used to detect the antigen.

In another format Multi-Antigenic Peptide (MAP) or similar antigen could be attached to a solid phase, and flagella antigen detected through its capacity to inhibit binding of immunoglobulin to MAP, by competition of MAP and flagella antigen for binding sites of the immunoglobulin.

Alternatively Multi-Antigenic Peptide (MAP) or similar antigen could be attached to a solid phase and two antibodies would be set in competition for binding to the MAP: a labelled anti-MAP antibody which does not bind to flagellin (the inventors have produced a monoclonal antibody with such a specificity as a by-product of preparing the 5836.1 hybridoma); and a MAb, which binds both MAP and flagellin. In the presence of flagellin antigen, the binding site of the MAb would be occupied and unable to compete with the MAP-specific antibody for binding to the solid phase. The consequence would be a rise in the ELISA signal in the presence of flagellin antigen.

The foregoing formats may generally be considered as ELISA-type detection methods. A different format would be the use of an immunochromatographic device, using similar apparatus to the Unipath CLEARVIEW™ device.

For example, a MAb could be used as either the capture antibody (nitrocellulose-linked) or the detection antibody (latex-linked) in the "CLEARVIEW"™ format for detection of flagellin antigen. A second antibody would be required to fulfil the other role. The second antibody would preferably be capable of binding all the *Salmonella* strains bound by the MAb and might additionally bind some Enterobacterial strains not bound by the MAb. The overall specificity, however, would remain that of the MAb.

Finally, the MAb could be used to detect *Salmonella* flagellin antigen in Western blots of bacterial proteins by standard protocols.

### Example 4

### Detection of Food-Borne Salmonella Using MAb from hybridoma 5836.1

A food sample would be mixed with a pre-enrichment medium and incubated for a period, typically overnight, at a temperature conducive to the recovery and growth of *Salmonella* cells (e.g. 35-37 C). A portion of the pre-enrichment broth would then be added to selective medium and incubated for a period, typically overnight, at a temperature favouring growth of *Salmonella* over competitor organisms (e.g. 35-37 C, or 43 C). A portion of the selection broth would then be added to a post-enrichment broth and incubated for a period, typically 6 hours, at a temperature conducive to the expression of flagellin antigen (e.g. 37 C).

Conceivably, the post-enrichment broth could be omitted and/or the pre-enrichment and selective media combined to streamline the culturing process. Following such traditional or streamlined culture of the organisms, a sample of the culture would be treated, typically by heating at 100 C for 10 minutes, but conceivably otherwise by acid, alkali or other chemical treatment, in order to expose the epitopes recognised by the antibodies in the test.

The presence of *Salmonella* flagella antigen in the sample would be revealed using an assay format such as that described in Example 3 (ideally by ELISA or use of an immunochromatograph device) employing a MAb such as that obtainable from hybridoma 5836.1. A positive reaction in the assay would indicate the presence of *Salmonella* organisms in the original food sample. A negative result would indicate the absence of *Salmonella,* or their presence below a certain threshold value.

## Claims

1. An immunoglobulin molecule or antigen-binding variant thereof which binds to an epitope present within amino acid residues 403 to 452 of the flagellin protein of *Salmonella typhimurium* PIR accession No. A24262, or the equivalent portion from other *Salmonella* strains.

2. A polyclonal or monoclonal antibody according to claim 1.

3. An immunoglobulin molecule or antigen-binding variant thereof according to claim 1 or 2, which binds to an epitope present within amino acid residues 418 to 452 of the flagellin protein of *Salmonella typhimurium* PIR accession No. A24262, or the equivalent portion from other *Salmonella* strains.

4. An immunoglobulin molecule or antigen-binding variant thereof according to any one of claims 1, 2 or 3 which binds to an epitope present within amino acid residues 427 to 436 of the flagellin protein of *Salmonella typhimurium* PIR accession No. A24262, or the equivalent portion from other *Salmonella* strains.

5. An immunoglobulin molecule or antigen-binding variant thereof according to any one of the preceding claims, which binds to the amino acid sequence RFDSAITNLG and/or RFNSAITNLG.

6. A monoclonal antibody according to any one of the preceding claims, obtainable from the hybridoma 5836.1.

7. An immunoglobulin molecule or antigen-binding variant thereof according to any one of claims 1, 2 or 3, which binds to an epitope present within amino acid residues 444 to 451 of the flagellin protein of *Salmonella typhimurium* PIR accession No. A24262, or the equivalent portion from other strains.

8. An immunoglobulin or antigen-binding variant thereof molecule according to any one of claims 1, 2 or 3, which binds to the amino acid sequence SARSRIED.

9. A monoclonal antibody according to claim 7 or 8, obtainable from the hybridoma 6035.1.

10. A monoclonal antibody according to any one of the preceding claims, present in a cell culture supernatant, ascites fluid or as a purified composition.

11. A host cell capable of producing an immunoglobulin molecule or antigen-binding variant thereof according to any one of the preceding claims.

12. A host cell according to claim 11, wherein the cell is an immortalised mammalian cell line.

13. A host cell according to claim 10 or 11, wherein the host cell is a hybridoma.

14. A host cell according to any one of claims 11, 12 or 13, being the hybridoma 5836.1 or 6035.1.

15. A nucleic acid sequence encoding an immunoglobulin molecule or antigen-binding variant thereof in accordance with any one of claims 1 to 10.

16. A nucleic acid sequence according to claim 15, obtainable from the hybridoma 5836.1 or 6035.1.

17. A method of producing an immunoglobulin molecule or antigen-binding variant thereof, the method comprising immunising a mammal with an immunogen which comprises at least one B-cell epitope present within amino acid residues 403 to 452 of the flagellin protein of *Salmonella typhimurium* PIR accession No. A24262, or the equivalent portion of flagellin from other *Salmonella* strains, said B-cell epitope being in isolation from *Salmonella* flagellin residues outside the portion 403 to 452.

18. A method according to claim 17, to produce a monoclonal antibody according to any one of claims 1 to 10.

19. A method according to claim 17 or 18, wherein the immunogen is a synthetic peptide.

20. A method of producing an immunoglobulin molecule or antigen-binding variant thereof, the method comprising the steps of: obtaining a library of nucleic acid sequences encoding immunoglobulin molecules or antigen-binding variants thereof; screening the library with a screening molecule which comprises at least a portion of amino acid residues 403 to 452 of the flagellin protein of *Salmonella typhimurium* PIR accession No. A 24262, or the equivalent portion of flagellin from other *Salmonella* strains, said portion being in substantial isolation from *Salmonella* flagellin residues outside the portion 403 to 452; selecting those nucleic acid sequences which encode an immunoglobulin or antigen-binding variant which binds to the screening molecule; and causing expression of the selected nucleic acid sequences to obtain the desired immunoglobulin or antigen-binding variant thereof.

21. A method for detecting the presence of *Salmonella* organisms in a sample under test, comprising contacting the sample with an immunoglobulin molecule or antigen-binding variant thereof which binds to an epitope present within amino acid residues 403 to 452 of the flagellin protein of *Salmonella* typhimurium PIR accession No. A24262, or the equivalent portion of flagellin from other *Salmonella* strain; and monitoring binding of the immunoglobulin molecule, or antigen-binding variant thereof, to the sample.

22. A method according to claim 21, comprising use of an immunoglobulin or antigen-binding variant thereof in accordance with any one of claims 1 to 10.

23. A method according to claim 21 or 22, wherein the anti-*Salmonella* immunoglobulin or antigen-binding variant thereof is labelled.

24. A method according to claim 21 or 22, comprising use of a second immunoglobulin or antigen-binding variant thereof to monitor binding of the anti-*Salmonella* immunoglobulin or antigen-binding variant to the sample under test.

25. A method according to claim 24, wherein the second immunoglobulin or antigen-binding variant has binding specificity for the anti-*Salmonella* immunoglobulin or variant thereof.

26. A method according to claim 24, wherein the second immunoglobulin or antigen-binding variant has anti-*Salmonella* binding specificity.

27. A method according to any one of claims 21-24, comprising use of an immunoglobulin molecule or antigen-binding variant thereof in accordance with claim 4, and use of an immunoglobulin molecule or antigen-binding variant thereof in accordance with claim 7.

28. A method according to any one of claims 21 to 27, comprising use of a label selected from the group consisting of: radioactive labels; enzyme labels; biochemical labels; and coloured latex particles.

29. An assay device for detecting the presence of *Salmonella* organisms in a sample under test, the device comprising an immunoglobulin molecule or antigen-binding variant thereof in accordance with any one of claims 1 to 10.

30. A device according to claim 29, for performing the method of any one of claims 21 to 28.

31. A molecule comprising at least part of amino acid residues 403 to 452 of the flagellin protein of *S. typhimurium* PIR accession No. A24262, or the equivalent portion from other *Salmonella* strains, in substantial isolation from other portions of the flagellin sequence.

32. A molecule according to claim 31, comprising residues 427 to 436 of the flagellin protein of *S. typhimurium* PIR accession No. A24262, or the equivalent portion from other *Salmonella* strains.

33. A molecule according to claim 31 or 32, comprising residues 444 to 451 of the flagellin protein of *S.* *typhimurium* PIR accession No. A24262, or the equivalent portion from other *Salmonella* strains.

34. A method of detecting the presence of *Salmonella* organisms in a sample under test, comprising use of a molecule comprising at least part of amino acid residues 403 to 452 of the flagellin protein of *S. typhimurium* PIR accession No. A24262, or the equivalent portion from other *Salmonella* strains, in substantial isolation from other portions of the flagellin sequence.

35. An assay device for detecting the presence of *Salmonella* organisms in a sample under test, the device comprising a molecule in accordance with any one of claims 31, 32 or 33.
